# EUROPEAN PATENT SPECIFICATION

(11) **EP 3 213 790 B1**
(45) Date of publication and mention of the grant of the patent: **28.11.2018**
(21) Application number: 15853902.3
(22) Date of filing: 17.09.2015
(51) Int. Cl.: A61M 25/09, A61M 25/00, A61M 25/01

(54) **BALLOON CATHETER**
BALLONKATHETER
CATHÉTER À BALLONNET

(30) Priority: 28.10.2014 JP 2014219250
(43) Date of publication of application: 06.09.2017
(73) Proprietor: Japan Lifeline Co., Ltd., Tokyo 140-0002 (JP)
(72) Inventor: OKAWA, Yasuhiro, Tokyo 140-0002 (JP)
(74) Representative: Grünecker Patent- und Rechtsanwälte PartG mbB
(86) International application number: PCT/JP2015/076594
(87) International publication number: WO 2016/067790

(56) References cited:
- EP-A1- 2 495 006
- WO-A1-2014/141440
- JP-A- 2001 029 449
- JP-A- 2003 517 901
- JP-A- 2008 503 249
- JP-A- 2008 536 639
- JP-A- 2012 020 077
- JP-A- 2012 020 077
- JP-A- 2014 195 487

## Description

### Technical Field

The present invention relates to a balloon catheter, and more specifically, it relates to a rapid exchange type balloon catheter that can be suitably used for the treatment of peripheral arterial disease by a crossover approach.

### Background Art

Hitherto, a balloon catheter having an outer shaft and an inner tube has been known (see, for example, PTL 1 and PTL 2).

In a rapid exchange type balloon catheter such as those shown in PTL 1 and PTL 2, a balloon is attached to the distal end of an outer shaft, the distal end of an inner tube is fixed to the distal end of the balloon, and the rear end of the inner tube opens on the side surface of the outer shaft and forms a guide wire port.

A rear end side shaft composed of a metal tube is connected to the rear end of the outer shaft composed of a resin tube.

The lumens of the outer shaft (distal end side shaft) and the rear end side shaft are expansion lumens through which fluid for expanding the balloon flows, and the lumen of the inner tube is a guide wire lumen for inserting a guide wire.

In a rapid exchange type balloon catheter such as those shown in PTL 1 and PTL 2, a core wire is inserted as a reinforcing body into the lumen of the outer shaft composed of a resin tube.

The rear end of the core wire is fixed by welding or the like to the inner peripheral surface of the rear end shaft composed of a metal tube. Thereby, the stiffness of the rear end part of the outer shaft (distal end side shaft) which needs to be reinforced, or more specifically, the part on the rear end side of the position where the guide wire port is formed can be improved, and the occurrence of kinking (buckling) of that part during the introduction of this balloon catheter can be suppressed to some extent.

The core wire generally has a straight portion having a constant diameter and a taper portion (reduced diameter portion) located on the distal end side of the straight portion. The taper portion of the core wire becomes smaller in diameter toward the distal end, and the hardness (stiffness) of the balloon catheter can thereby be gradually reduced toward the distal end.

However, in the balloon catheter such as those shown in PTL 1 and PTL 2, the distal end part of the core wire is not fixed to the inner peripheral surface of the outer shaft (the distal end is a free end), and therefore, there is a problem in that sufficient pushability cannot be exhibited.

That is, when pushing force is applied from the rear end side shaft when inserting this balloon catheter into a blood vessel, the core wire moves (advances) in the axial direction relative to the outer shaft (distal end side shaft), and the pushing force cannot be sufficiently transmitted to the distal end side shaft.

In the balloon catheter in which the distal end of the core wire is not fixed to the inner peripheral surface of the outer shaft, when inserting into a blood vessel (when pushing force is applied), kinking of the rear end part of the distal end side shaft (the part from the position where the guide wire port is formed to the position of the distal end of the rear end side shaft) cannot be reliably prevented.

To solve such a problem, PTL 3 discloses a balloon catheter in which, in the rear end part of an outer shaft (distal end side shaft), a core wire is fixed to the inner peripheral surface of the outer shaft.

By fixing the core wire fixed to the inner peripheral surface of the rear end side shaft also to the inner peripheral surface of the distal end side shaft, like the balloon catheter described in PTL 3, the pushing force from the rear end side shaft can be reliably transmitted to the distal end side shaft, and good pushability can be exhibited. In addition, the stiffness of the distal end side shaft on the rear end side of the position where the core wire is fixed can be sufficiently increased, and kinking in that part can be reliably prevented.

However, when the core wire is firmly fixed to the inner peripheral surface of the distal end side shaft, like the balloon catheter described in PTL 3, the bending stiffness of the distal end side shaft is too high, and flexibility is significantly impaired. As a result, a problem arises in that such a balloon catheter cannot be smoothly inserted into a curved blood vessel.

When the position where the core wire is fixed on the inner peripheral surface of the distal end side shaft is significantly spaced to the rear end side from the position where the guide wire port is formed, the stiffness of the part from the position where the guide wire port is formed to the position where the core wire is fixed cannot be sufficiently increased, and therefore, kinking may occur in that part.

Now, a femoral artery approach (crossover approach) in which the femoral artery on the side opposite to the lesion is punctured and a balloon catheter is inserted, and the balloon catheter is brought to the femoral artery on the side of the lesion through the iliac artery, has been selected as a recent treatment of peripheral arterial disease.

However, the balloon catheter such as those described in PTLs 1 to 3 has a rear end side shaft composed of a metal tube, and is therefore difficult to insert into a curved blood vessel leading from the femoral artery on the side opposite to the lesion through the iliac artery to the femoral artery on the side of the lesion. That is, in this case, the balloon catheter needs to be passed through a sharply curved blood vessel, but since the metal tube has a too high stiffness compared to the resin tube, the balloon catheter may kink at the junction between the outer tube and the metal tube when passing through that curve. Therefore, the conventional balloon catheter is difficult to use in a procedure that uses a crossover approach.

A further balloon catheter having an outer shaft, a balloon, and an inner tube, wherein between the inner tube and the outer shaft a stiffening wire is provided, is known from JP 2012 020077A. Features of the preamble of claim 1 are known from this document.

### Patent Literature

PTL 1: Japanese Unexamined Patent Application Publication No. 2002-28243
PTL 2: Japanese Unexamined Patent Application Publication No. 2003-164528
PTL 3: Japanese Patent No. 5061614

### Summary of Invention

### Technical Problem

The present invention has been made in view of the above circumstances.

An object of the present invention is to provide a rapid exchange type balloon catheter that has excellent pushability upon insertion into a blood vessel, and in which kinking (buckling) of an outer shaft on the rear end side of a position where a guide wire port is formed can be reliably prevented, and that can be smoothly inserted even into a curved blood vessel.

Another object of the present invention is to provide a rapid exchange type balloon catheter that can be used also in a procedure that uses a crossover approach.

### Solution to Problem

(1) A balloon catheter of the present invention includes
   an outer shaft composed of a resin tube,
   a balloon connected to the distal end of the outer shaft,
   a hub connected to the rear end of the outer shaft,
   an inner tube that is a resin tube inserted into the lumen of the outer shaft and the inside of the balloon and forming a guide wire lumen, the rear end of which opens as a guide wire port on the side surface of the outer shaft, the distal end portion of which is fixed to the distal end portion of the balloon, and the distal end of which opens, and
   a core wire that has a straight portion and a reduced diameter portion and that is inserted into the lumen of the outer shaft with the reduced diameter portion on the distal end side of the straight portion.

The core wire is fixed to the inner peripheral surface of the outer shaft on the rear end side of the position where the guide wire port is formed, and the straight portion of the core wire is press-fitted between the inner peripheral surface of the outer shaft and the outer peripheral surface of the inner tube.

The distance (L1) in the axial direction from the position where the guide wire port is formed to the distal end of the straight portion of the core wire is 1.0 to 50.0 mm.

The cross-section of a part of the outer shaft in which the straight portion is press-fitted is deformed into an elliptical shape by the press-fitting of the straight portion of the core wire, and assuming that the length of the major axis of the ellipse is denoted as (D₁₁), and the length of the minor axis of the ellipse is denoted as (D₁₂), the value of (D₁₁)/(D₁₂) is 1.02 to 1.30.

Since, in a balloon catheter having such a configuration, the straight portion of the core wire fixed to the inner peripheral surface of the outer shaft on the rear end side of the position where the guide wire port is formed, is fixed by press-fitting between the inner peripheral surface of the outer shaft and the outer peripheral surface of the inner tube, excellent pushability can be exhibited. In addition, since the stiffness of the outer shaft on the rear end side of the position where the core wire is fixed (the region including the part from the position where the guide wire port is formed to the position where the core wire is fixed to the inner peripheral surface) can be sufficiently increased, kinking of the outer shaft on the rear end side of the position where the guide wire port is formed can be reliably prevented.

Since the straight portion is press-fitted between the inner peripheral surface of the outer shaft and the outer peripheral surface of the inner tube, and the core wire is thereby fixed (so to speak, half-fixed by utilizing the elasticity of resin), when the outer shaft is subjected to bending stress, the core wire can be moved (slid) in the axial direction relative to the outer shaft. Therefore, the outer shaft has good flexibility. As a result, the outer shaft can be smoothly inserted into a curved blood vessel.

Since the outer shaft is composed of a resin tube throughout its length, this balloon catheter can be inserted even into a curved blood vessel leading from the femoral artery on the side opposite to the lesion through the iliac artery to the femoral artery on the side of the lesion, and can be suitably used in a procedure that uses a crossover approach.
(2) In the balloon catheter of the present invention, it is preferable that, assuming that the inside diameter of the resin tube composing the outer shaft is denoted as (d₁), the outside diameter of the inner tube is denoted as (D₂), and the diameter of the straight portion of the core wire is denoted as (D₃), the value of D₃/(d₁-D₂) be 1.02 to 1.70. However, the inside diameter (d₁) of the resin tube is the inside diameter in a state where the core wire is not inserted.
   In a balloon catheter having such a configuration, moderate pressure (sandwiching force) can be applied to the outer peripheral surface of the straight portion by the inner peripheral surface of the outer shaft and the outer peripheral surface of the inner tube. Therefore, this outer shaft has balanced excellent stiffness and flexibility.
(3) In the balloon catheter of the present invention, it is preferable that a strain relief be provided at the junction between the rear end of the outer shaft and the hub, and the rear end of the core wire be located inside the strain relief.
   With a balloon catheter having such a configuration, kinking can be prevented in the whole thereof.
(4) It is preferable that, in the balloon catheter of the present invention, the distance (L4) in the axial direction from the position where the guide wire port is formed to the position where the core wire is fixed to the inner peripheral surface of the outer shaft (if the core wire is continuously fixed, the position of the distal end of the fixation part) be less than or equal to 600 mm.
   In a balloon catheter having such a configuration, the occurrence of kinking in the part from the position where the guide wire port is formed to the position where the core wire is fixed can be effectively prevented.

### Advantageous Effects of Invention

The balloon catheter of the present invention has excellent pushability upon insertion into a blood vessel. In the balloon catheter of the present invention, kinking (buckling) of an outer shaft on the rear end side of a position where a guide wire port is formed can be reliably prevented, and the balloon catheter of the present invention can be smoothly inserted even into a curved blood vessel.

The balloon catheter of the present invention can be suitably used also in a procedure that uses a crossover approach.

### Brief Description of Drawings

[Fig. 1] Fig. 1 is a sectional view schematically showing a longitudinal section of a balloon catheter according to an embodiment of the present invention.
[Fig. 2] Fig. 2 includes sectional views showing cross-sections of the balloon catheter according to an embodiment of the present invention (sectional views taken along lines a-a, b-b, c-c, d-d, and e-e of Fig. 1).

### Description of Embodiments

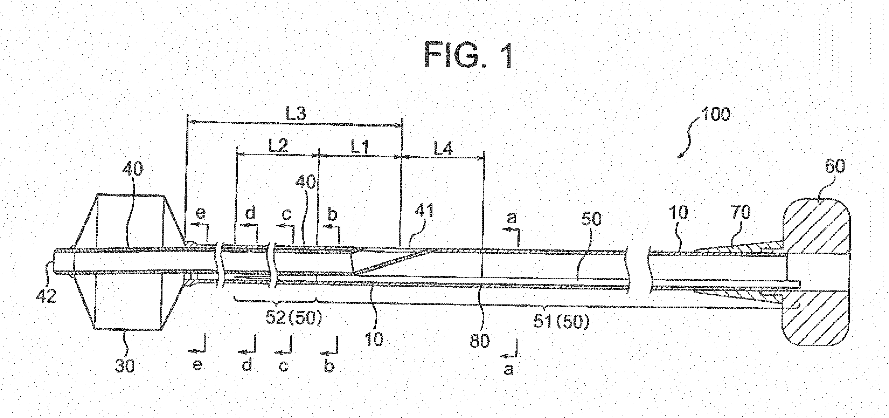

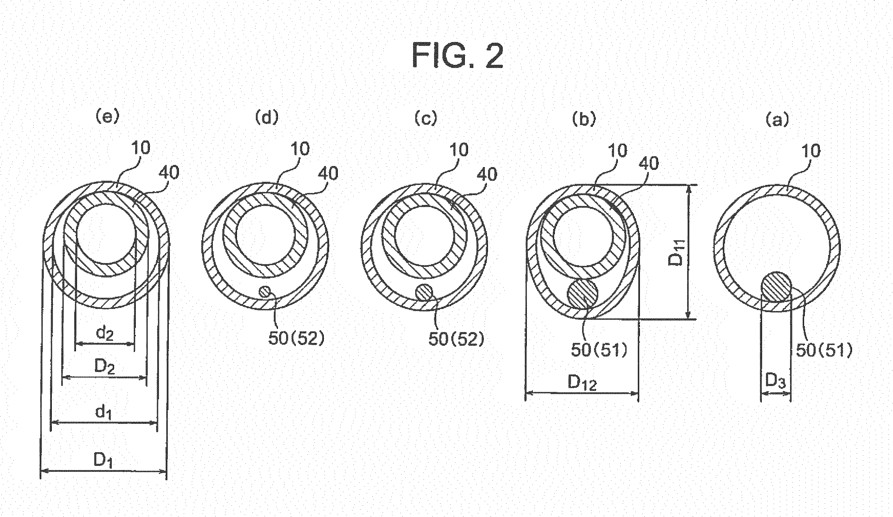

A balloon catheter 100 of this embodiment shown in Fig. 1 and Fig. 2 is used in the treatment (procedure) of peripheral arterial disease (PAD) of the lower extremities.

The balloon catheter 100 includes an outer shaft 10 composed of a resin tube, a balloon 30 connected to the distal end of the outer shaft 10, a hub 60 connected to the rear end of the outer shaft 10, an inner tube 40 that is a resin tube inserted into the lumen of the outer shaft 10 and the inside of the balloon 30 and forming a guide wire lumen, the rear end of which opens as a guide wire port on the side surface of the outer shaft 10, the distal end portion of which is fixed to the distal end portion of the balloon 30, and the distal end of which opens, and a core wire 50 that consists of a straight portion 51 and a taper portion 52 (reduced diameter portion) and that is inserted into the lumen of the outer shaft 10 with the taper portion 52 on the distal end side. The rear end side of the straight portion 51 of the core wire 50 is fixed to the inner peripheral surface of the outer shaft 10 on the rear end side of the position where the guide wire port is formed, and the distal end side of the straight portion 51 of the core wire 50 is press-fitted between the inner peripheral surface of the outer shaft 10 and the outer peripheral surface of the inner tube 40. The distance (L1) in the axial direction from the position where the guide wire port is formed to the distal end of the straight portion 51 of the core wire 50 is 1.0 to 50.0 mm. The cross-section of a part of the outer shaft 10 in which the straight portion 51 is press-fitted is deformed into an elliptical shape by the press-fitting of the straight portion 51 of the core wire 50. Assuming that the length of the major axis of the ellipse is denoted as (D₁₁), and the length of the minor axis of the ellipse is denoted as (D₁₂), the value of (D₁₁)/(D₁₂) is 1.02 to 1.30.

The outer shaft 10 of the balloon catheter 100 is composed of a resin tube throughout its length from the junction with the balloon 30 to the junction with the hub 60.

A lumen (expansion lumen) through which fluid for expanding the balloon 30 flows is formed in the outer shaft 10.

The outside diameter (D₁) of the resin tube composing the outer shaft 10 is generally 0.8 to 1.4 mm.

The inside diameter (d₁) of the resin tube composing the outer shaft 10 is generally 0.70 to 1.30 mm.

The outside diameter (D₁) and the inside diameter (d₁) are diameters in a state where the core wire 50 is not inserted.

Constituent materials of the outer shaft 10 (resin tube) include thermoplastic resins such as polyamide, polyether polyamide, polyurethane, polyether block amide (PEBAX) (registered trademark), and nylon. PEBAX is the most preferable among these.

The hardness of the outer shaft 10 (resin tube) is preferably 63 to 80 as measured by use of a D-type durometer.

The outer shaft 10 may be formed of a resin having the same hardness along the axial direction, or may be integrally formed of resins having different hardnesses along the axial direction.

The balloon 30 is connected to the distal end of the outer shaft 10.

The balloon 30 is expanded by liquid flowing through the lumen of the outer shaft 10. Liquids include saline and contrast agent.

The same constituent materials as those of the balloon composing the conventional known balloon catheter can be used as constituent materials of the balloon 30. PEBAX can be cited as a preferable material.

On the other hand, the hub 60 is connected to the rear end of the outer shaft 10 with a strain relief 70 interposed therebetween.

The inner tube 40 composing the balloon catheter 100 is a resin tube that extends in the lumen of the outer shaft 10 and the inside (inner cavity) of the balloon 30 and forms a lumen for inserting a guide wire (guide wire lumen).

The rear end of the inner tube 40 opens on the side surface of the outer shaft 10, and this opening 41 serves as a guide wire port.

The distal end portion of the inner tube 40 is fixed to the distal end portion of the balloon 30, and an opening 42 is formed at the distal end of the inner tube 40.

The outside diameter (D₂) of the inner tube 40 is generally 0.48 to 0.60 mm.

The inside diameter (d₂) of the inner tube 40 is generally 0.35 to 0.45 mm.

The distance from the position where the opening 41 of the inner tube 40 serving as a guide wire port is formed to the distal end of the balloon 30 is 200 to 400 mm. The distance (L3) in the axial direction from the position where the opening 41 is formed to the position of the rear end of the balloon 30 can be appropriately adjusted to the length of the balloon 30.

Constituent materials of the inner tube 40 include the same synthetic resins as constituent materials of the outer shaft 10. PEBAX is the most preferable among these.

The hardness of the outer shaft 10 is preferably 63 to 80 as measured by use of a D-type durometer.

The core wire 50 composing the balloon catheter 100 consists of a straight portion 51 and a taper portion 52. The core wire 50 is inserted into the lumen (expansion lumen) of the outer shaft 10 with the taper portion 52 on the distal end side.

As shown in Fig. 1, the rear end of the core wire 50 reaches the inside of the hub 60. Thereby, although the outer shaft 10 is composed of a resin tube throughout its length (although the rear end side is not composed of a metal tube), the stiffness on the rear end side of the outer shaft 10 can be sufficiently secured.

The rear end of the core wire 50 may be located inside the strain relief 70. The reason is that when the core wire 50 extends to the inside of the strain relief 70, kinking can be prevented in the whole balloon catheter 100.

The rear end side of the straight portion 51 of the core wire 50 is fixed to the inner peripheral surface of the outer shaft 10 (the inner peripheral surface on the rear end side of the position where the opening 41 serving as a guide wire port is formed).

"The rear end side of the straight portion 51" means the rear end side relative to "the distal end side of the straight portion 51" press-fitted between the outer shaft 10 and the inner tube 40.

The method for fixing the core wire 50 to the inner peripheral surface of the outer shaft 10 is not particularly limited as long as a firmly fixed state can be achieved. Examples thereof include a fixing method using adhesive, and a method in which a resin thin film is closely attached to the surface of the core wire 50 and this resin thin film fused to the inner peripheral surface of the outer shaft 10.

The fixation of the core wire 50 to the inner peripheral surface of the outer shaft 10 may be performed continuously along the axial direction or may be performed in a spot manner.

The core wire 50 "fixed" to the inner peripheral surface of the outer shaft 10 does not move in the axial direction from the fixation position when the outer shaft 10 is subjected to bending stress.

The distance (L4) in the axial direction from the position where the opening 41 (guide wire port) of the inner tube 40 is formed to the position where the core wire 50 is fixed to the inner peripheral surface of the outer shaft 10 (fixation position 80 shown in Fig. 1) is preferably less than or equal to 600 mm, more preferably 1 to 150 mm.

The fixation position 80 when the core wire 50 is continuously fixed to the inner peripheral surface of the outer shaft 10 is the position of the distal end of the fixed part.

When this distance (L4) is too long, the stiffness of the part from the position where the opening 41 is formed to the fixation position 80 of the core wire 50 cannot be sufficiently increased, and therefore, kinking may occur in that part.

The distal end side of the straight portion 51 of the core wire 50 is fixed to the outer shaft 10 by being press-fitted between the inner peripheral surface of the outer shaft 10 and the outer peripheral surface of the inner tube 40 (by sandwiching the straight portion 51 between the two resin tubes).

"The distal end side of the straight portion 51" means the distal end side relative to "the rear end side of the straight portion 51" fixed to the inner peripheral surface of the outer shaft 10.

Since, as described above, the straight portion 51 of the core wire 50 fixed to the inner peripheral surface of the outer shaft 10 on the rear end side of the position where the opening 41 (guide wire port) of the inner tube 40 is formed, is fixed by press-fitting between the inner peripheral surface of the outer shaft 10 and the outer peripheral surface of the inner tube 40, pushing force from the rear end side of the outer shaft 10 can be reliably transmitted to the distal end side, and good pushability can be exhibited.

Since the stiffness of the outer shaft 10 on the rear end side of the position where the core wire 50 is fixed (the region including the part from the position where the opening 41 is formed to the fixation position 80 of the core wire 50) can be sufficiently increased, kinking of the outer shaft 10 on the rear end side of the position where the opening 41 (guide wire port) is formed can be reliably prevented.

On the distal end side of the straight portion 51, the core wire 50 is not completely fixed to the inner peripheral surface of the outer shaft 10 but is fixed (half-fixed) by press-fitting the straight portion 51 between the inner peripheral surface of the outer shaft 10 and the outer peripheral surface of the inner tube 40. Therefore, when the outer shaft 10 is subjected to bending stress, the core wire 50 can be moved (slid) in the axial direction relative to the outer shaft 10, and therefore the outer shaft 10 has good flexibility. As a result, the outer shaft 10 can be smoothly inserted into a curved blood vessel.

When, instead of the straight portion 51, the taper portion 52 of the core wire 50 is press-fitted between the inner peripheral surface of the outer shaft 10 and the outer peripheral surface of the inner tube 40, the core wire 50 cannot be moved relative to the outer shaft 10 toward the distal end, and therefore, flexibility secured by the relative movement of the core wire 50 toward the distal end cannot be secured.

When the core wire 50 is moved relative to the outer shaft 10 toward the rear end, and the taper portion 52 is thereby released from the press-fitting fixation, the core wire 50 is no longer in contact with the outer shaft 10 and the inner tube 40. Therefore, good pushability cannot be exhibited, and the occurrence of kinking of the outer shaft 10 on the rear end side of the position where the opening 41 (guide wire port) is formed cannot be prevented.

The diameter (D₃) of the straight portion 51 of the core wire 50 varies depending on the inside diameter (d₁) of the resin tube composing the outer shaft 10 and the outside diameter (D₂) of the inner tube 40, and at least needs to be adjusted such that the value of D₃/(d₁-D₂) is greater than 1.0. The diameter (D₃) of the straight portion 51 is generally 0.1 to 0.3 mm.

The diameter (D₃) of the straight portion 51 is preferably adjusted such that the value of D₃/(d₁-D₂) is 1.02 to 1.70.

By adjusting the diameter (D₃) such that the value of D₃/(d₁-D₂) is 1.02 to 1.70, moderate pressure (sandwiching force) can be applied to the outer peripheral surface of such a straight portion 51 by the inner peripheral surface of the outer shaft 10 and the outer peripheral surface of the inner tube 40. As a result, the outer shaft 10 has balanced excellent stiffness and flexibility.

When the value of D₃/(d₁-D₂) is less than 1.02, the straight portion of the core wire cannot be sufficiently fixed to the outer shaft. On the other hand, when the value of D₃/(d₁-D₂) is greater than 1.70, the straight portion of the core wire may not be able to be press-fitted between the inner peripheral surface of the outer shaft and the outer peripheral surface of the inner tube, or the cross-sectional shape of the shaft after press-fitting fixation may be significantly deformed, and the operability may be impaired.

As shown in Fig. 2 (b), in the balloon catheter 100 of this embodiment, the cross-section of a part of the outer shaft 10 to which the straight portion 51 of the core wire 50 is fixed by press-fitting is deformed from a circular shape before press-fitting to an elliptical shape.

Assuming that the length of the major axis of the ellipse is denoted as (D₁₁), and the length of the minor axis of the ellipse is denoted as (D₁₂), the value of (D₁₁)/(D₁₂) is generally 1.02 to 1.30, preferably 1.02 to 1.15.

Since the value of (D₁₁)/(D₁₂) in the above ellipse is 1.02 to 1.30, the matching property of the outer shaft 10 with other devices is not impaired, and the improving effect on the pushability, and the improving effect on the stiffness of the outer shaft on the rear end side of the position where the guide wire port is formed, of the present invention can be sufficiently exhibited.

When the value of (D₁₁)/(D₁₂) is less than 1.02, the straight portion of the core wire inserted into the lumen of the outer shaft is not sufficiently fixed by press-fitting, and therefore the improving effect on the pushability, and the improving effect on the stiffness of the outer shaft cannot be sufficiently exhibited, or the lumen of the inner tube (guide wire lumen) may be blocked.

On the other hand, when the value of (D₁₁)/(D₁₂) is greater than 1.30, the matching property of the outer shaft with other devices may be impaired. For example, the outer shaft having such a cross-sectional shape may not be able to be housed in a guiding sheath.

The distance (L1) in the axial direction from the position where the opening 41 of the inner tube 40 serving as a guide wire port is formed to the distal end of the straight portion 51 of the core wire 50 is generally 1.0 to 50.0 mm.

when this distance (L1) is too short, the core wire 50 (straight portion 51) cannot be sufficiently fixed (sandwiched), and the improving effect on the pushability, and the improving effect on the stiffness of the outer shaft cannot be sufficiently exhibited.

On the other hand, when this distance (L1) is too long, "directivity of bending" appears in the outer shaft 10, and the operability (ease of insertion into a blood vessel) of the balloon catheter is impaired.

The length (L2) of the taper portion 52 of the core wire 50 varies depending, for example, on the distance (L3) in the axial direction from the position where the opening 41 is formed to the position of the rear end of the balloon 30, and is generally 50 to 250 mm.

The balloon catheter 100 of this embodiment has excellent pushability upon insertion into a blood vessel. In this balloon catheter 100, kinking of the outer shaft 10 on the rear end side of the position where the opening 41 (guide wire port) is formed (in particular, the part from the position where the opening 41 is formed to the fixation position 80 of the core wire 50) can be reliably prevented, and since the outer shaft 10 has moderate flexibility, this balloon catheter 100 can be smoothly inserted even into a curved blood vessel.

Since the outer shaft 10 is composed of a resin tube throughout its length and a metal tube is not used, this balloon catheter 100 can be inserted even into a curved blood vessel leading from the femoral artery on the side opposite to the lesion through the iliac artery to the femoral artery on the side of the lesion, and can be suitably used in a procedure that uses a crossover approach.

Although an embodiment of the present invention has been described, the present invention is not limited to this, and various changes may be made within the scope of the appended claims.

For example, the taper portion (reduced diameter portion) of the core wire may be reduced in diameter intermittently toward the distal end.

In the core wire a plurality of straight portions (portions having a constant diameter) having different diameters may be connected to each other with reduced diameter portions interposed therebetween. In this case, any one of the straight portions is press-fitted between the inner peripheral surface of the outer shaft and the outer peripheral surface of the inner tube.

### Reference Signs List

- 100: balloon catheter
- 10: outer shaft
- 30: balloon
- 40: inner tube
- 41: opening (guide wire port)
- 42: opening
- 50: core wire
- 51: straight portion
- 52: taper portion
- 60: hub
- 70: strain relief
- 80: fixation position

## Claims

1. A balloon catheter (100) comprising:
an outer shaft (10) composed of a resin tube;
a balloon (30) connected to the distal end of the outer shaft (10);
a hub (60) connected to the rear end of the outer shaft (10);
an inner tube (40) that is a resin tube inserted into the lumen of the outer shaft (10) and the inside of the balloon (30) and forming a guide wire lumen, the rear end of which opens as a guide wire port (41) on the side surface of the outer shaft (10), the distal end portion of which is fixed to the distal end portion of the balloon (30), and the distal end of which opens; and
a core wire (50) that has a straight portion (51) and a reduced diameter portion (52) and that is inserted into the lumen of the outer shaft (10) with the reduced diameter portion on the distal end side of the straight portion (51),
**characterized in that** the core wire (50) is fixed to the inner peripheral surface of the outer shaft (10) on the rear end side of the position where the guide wire port (41) is formed, and the straight portion (51) of the core wire is press-fitted between the inner peripheral surface of the outer shaft (10) and the outer peripheral surface of the inner tube (40),
wherein the distance (L1) in the axial direction from the position where the guide wire port (41) is formed to the distal end of the straight portion (51) of the core wire is 1.0 to 50.0 mm, and
wherein the cross-section of a part of the outer shaft (10) in which the straight portion (51) is press-fitted is deformed into an elliptical shape by the press-fitting of the straight portion (51) of the core wire (50), and assuming that the length of the major axis of the ellipse is denoted as (D₁₁), and the length of the minor axis of the ellipse is denoted as (D₁₂), the value of (D₁₁)/(D₁₂) is 1.02 to 1.30.

2. The balloon catheter (100) according to Claim 1, wherein assuming that the inside diameter of the resin tube composing the outer shaft (10) is denoted as (d₁), the outside diameter of the inner tube (40) is denoted as (D₂), and the diameter of the straight portion (51) of the core wire is denoted as (D₃), the value of D₃/(d₁-D₂) is 1.02 to 1.70.

3. The balloon catheter (100) according to Claim 1 or 2, wherein a strain relief (70) is provided at the junction between the rear end of the outer shaft (10) and the hub (60), and the rear end of the core wire (50) is located inside the strain relief (70).

## Patentansprüche

1. Ballonkatheter (100) umfassend:
einen äußeren Schaft (10), die aus einem Harzrohr aufgebaut ist;
ein Ballon (30), der mit dem distalen Ende des äußeren Schafts (10) verbunden ist;
eine Nabe (60), die mit dem hinteren Ende des äußeren Schafts (10) verbunden ist;
ein inneres Rohr (40), das ein Harzrohr ist, das in das Lumen des äußeren Schafts (10) und das Innere des Ballons (30) eingesetzt ist und ein Führungsdrahtlumen bildet, dessen hinteres Ende sich als Führungsdrahtöffnung (41) auf der Seitenfläche des äußeren Schafts (10) öffnet, dessen distaler Endabschnitt an dem distalen Endabschnitt des Ballons (30) befestigt ist und dessen distales Ende sich öffnet; und
einen Kerndraht (50), der einen geraden Abschnitt (51) und einen Abschnitt (52) mit verringertem Durchmesser aufweist und der in das Lumen des äußeren Schafts (10) mit dem Abschnitt mit reduziertem Durchmesser an der distalen Endseite des geraden Abschnitts (51) eingeführt ist,
**dadurch gekennzeichnet,**
**dass** der Kerndraht (50) an der inneren Umfangsfläche des äußeren Schafts (10) an der hinteren Endseite der Position, an der die Führungsdrahtöffnung (41) ausgebildet ist, fixiert ist, und der gerade Abschnitt (51) des Kerndrahts (50) zwischen der innere Umfangsfläche des äußeren Schafts (10) und der äußere Umfangsfläche der inneren Rohrs (40) pressgepasst ist,
wobei der Abstand (L1) in der axialen Richtung von der Position, an der die Führungsdrahtöffnung (41) ausgebildet ist, zu dem distalen Ende des geraden Abschnitts (51) des Kerndrahtes 1,0 bis 50,0 mm beträgt, und
wobei der Querschnitt eines Teils des äußeren Schafts (10), in den der gerade Abschnitt (51) pressgepasst ist, durch das Presspassen des geraden Abschnitts (51) des Kerndrahts in eine elliptische Form verformt wird, wenn angenommen wird, dass die Länge der Hauptachse der Ellipse als (D11) bezeichnet ist und die Länge der Nebenachse der Ellipse als (D12) bezeichnet ist, der Wert von (D11) / (D12) 1,02 bis 1,30 beträgt.

2. Ballonkatheter (100) nach Anspruch 1, bei dem, wenn angenommen wird, dass der Innendurchmesser des Harzrohrs, das den äußeren Schaft (10) bildet, mit (d1) bezeichnet ist, der Außendurchmesser des inneren Rohrs (40) mit (D2) bezeichnet ist, und der Durchmesser des geraden Abschnitts (51) des Kerndrahtes mit (D3) bezeichnet ist, der Wert von D3/(d1-D2) 1,02 bis 1,70 beträgt.

3. Ballonkatheter (100) nach Anspruch 1 oder 2, bei dem eine Zugentlastung (70) an der Verbindungsstelle zwischen dem hinteren Ende des äußeren Schafts (10) und der Nabe (60) vorgesehen ist, und das hintere Ende des Kerndrahts (50) sich innerhalb der Zugentlastung (70) befindet.

## Revendications

1. Cathéter à ballonnet (100) comprenant :
une tige externe (10) composée d'un tube de résine ;
un ballonnet (30) relié à l'extrémité distale de la tige externe (10) ;
un raccord (60) relié à l'extrémité arrière de la tige externe (10) ;
un tube interne (40) qui est un tube de résine inséré dans la lumière de la tige externe (10) et l'intérieur du ballonnet (30) et formant une lumière fil guide, dont l'extrémité arrière s'ouvre comme un orifice de fil guide (41) sur la surface latérale de la tige externe (10), dont la portion d'extrémité distale est fixée à la portion d'extrémité distale du ballonnet (30), et dont l'extrémité distale s'ouvre ; et
un fil formant noyau (50) qui présente une portion droite (51) et une portion de diamètre réduit (52) et qui est inséré dans la lumière de la tige externe (10) avec la portion de diamètre réduit sur le côté d'extrémité distale de la portion droite (51),
**caractérisé en ce que**
le fil formant noyau (50) est fixé à la surface périphérique interne de la tige externe (10) sur le côté d'extrémité arrière de la position où l'orifice de fil guide (41) est formé, et la portion droite (51) du fil formant noyau est ajustée par pression entre la surface périphérique interne de la tige externe (10) et la surface périphérique externe du tube interne (40),
la distance (L1) dans le sens axial depuis la position où l'orifice de fil guide (41) est formé vers l'extrémité distale de la portion droite (51) du fil formant noyau est de 1,0 à 50,0 mm, et
la section transversale d'une partie de la tige externe (10) dans laquelle la portion droite (51) est ajustée par pression est déformée en une forme elliptique par l'ajustement par pression de la portion droite (51) du fil formant noyau (50), et en faisant l'hypothèse que la longueur de l'axe majeur de l'ellipse est indiquée par (D₁₁), et que la longueur de l'axe mineur de l'ellipse est indiquée par (D₁₂), la valeur de (D₁₁)/(D₁₂) est de 1,02 à 1,30.

2. Cathéter à ballonnet (100) selon la revendication 1, dans lequel en faisant l'hypothèse que le diamètre interne du tube de résine composant la tige externe (10) est indiqué par (d₁), le diamètre externe du tube interne (40) est indiqué par (D₂), et le diamètre de la portion droite (51) du fil formant noyau est indiqué par (D₃), la valeur de D₃/(d₁-D₂) est de 1,02 à 1,70.

3. Cathéter à ballonnet (100) selon la revendication 1 ou 2, dans lequel un relâchement de tension (70) est fourni à la jonction entre l'extrémité arrière de la tige externe (10) et le raccord (60), et l'extrémité arrière du fil formant noyau (50) est localisée à l'intérieur du relâchement de tension (70).
